# NOUVEAU FASCICULE DE BREVET EUROPEEN

(11) **EP 0 607 090 B2**
(45) Date de publication et mention de la décision concernant l'opposition: **30.08.2000**
(45) Mention de la délivrance du brevet: 19.11.1997
(21) Numéro de dépôt: 94400091.8
(22) Date de dépôt: 14.01.1994
(51) Int. Cl.: A61F 13/15

(54) **Serviette périodique ou similaire à volets latéraux et procédé de fabrication**
Damenbinde mit Seitenteilen und Verfahren zu deren Herstellung
Sanitary towel or similar with side flaps and manufacturing method therefor

(30) Priorité: 15.01.1993 FR 9300367
(43) Date de publication de la demande: 20.07.1994
(73) Titulaire: FORT JAMES FRANCE, 68320 Kunheim (FR)
(72) Inventeur: Zauner, Jean-Luc, F-68420 Eguisheim (FR); Vandevelde, Herman, B-9880 Lotenhulle (BE)
(74) Mandataire: David, Daniel

(56) Documents cités:
- EP-A- 0 270 058
- WO-A-93/01785
- WO-A-94/14398
- GB-A- 2 233 235

## Description

La présente invention concerne une serviette périodique ou similaire à volets latéraux pour améliorer la tenue et l'étanchéïté latérale et son procédé de fabrication. Il est connu par le brevet américain US-A-4 701 178 une serviette périodique, ayant des extrémités opposées et des bords longitudinaux, comprenant : un élément absorbant central avec un côté faisant face au corps et un côté faisant face au vêtement. une feuille de matériau imperméable aux fluides disposée sur le côté faisant face au vêtement et ayant un côté faisant face à l'élément absorbant, et une paire de volets s'étendant respectivement à partir des bords longitudinaux de la serviette avec des côtés faisant face au corps et des côtés faisant face au vêtement. Des moyens adhésifs sont disposés sur les côtés de l'élément imperméable et d'au moins un des volets, faisant face au vêtement. Ces volets peuvent être ensuite repliés autour du vêtement de façon que la feuille imperméable l'entoure et le protège des fluides.

Ces serviettes périodiques nécessitent, dans le procédé de fabrication, deux postes de dépôt de l'adhésif : l'un sur la partie centrale de l'élément imperméable. l'autre sur les volets sur le côté faisant face au vêtement.

Cette opération de dépose est difficile à industrialiser et conduit à un produit d'emploi peu aisé pour l'utilisatrice. En effet, si les volets latéraux sont rabattus sur la bande siliconé recouvrant les zones adhésives disposées sur la partie centrale de la serviette, l'utilisatrice aura des difficultés à détacher les volets latéraux et ensuite la bande siliconée. L'invention a pour but de proposer une serviette périodique à volets latéraux adhésifs qui soit d'utilisation plus facile et également de fabrication plus aisée.

Ce but est atteint par la serviette périodique selon la revendication 1.

Selon une autre particularité. la serviette périodique comprend des moyens de protection constitués par une bande de papier siliconé. Selon une autre particularité, les volets latéraux sont constitués d'un matériau imperméable au fluide revêtu d'une matière absorbante.

Un autre but de l'invention est de proposer un procédé de fabrication de serviettes périodiques plus facile à mettre en oeuvre.

Ce but est atteint par le fait que le procédé de fabrication de serviettes périodiques selon l'invention comportant des étapes de formation de l'élément tampon associé avec un élément imperméable faisant face au vêtement et pourvu dans la zone centrale de volets latéraux, est caractérisé en ce qu'il comporte en outre :
- une étape de pliage des volets sur la zone centrale autour de lignes de pliage longitudinales en ménageant une bande centrale non recouverte par les volets ;
- une étape de dépôt de pavés de colle recouvrant la zone centrale et une portion de surface des volets tournés vers l'extérieur de la serviette périodique et
- une étape de mise en place d'un élément de protection des parties encollées.

Selon une autre particularité, le procédé comporte une étape de dépôt de pavés de colle à ses extrémités antérieure et postérieure.

Un autre but est de proposer une autre variante de procédé de fabrication plus facile à réaliser et permettant d'obtenir des serviettes périodiques pratiques à l'emploi.

Ce but est atteint par le fait que le procédé de fabrication des serviettes périodiques selon l'invention comporte des étapes de formation de l'élément tampon associé à l'élément faisant face au vêtement et pourvu dans la zone centrale de volets latéraux est caractérisé en ce qu'il comporte en outre une étape de pliage des volets sur la zone centrale en ménageant une bande centrale non recouverte par les volets, une étape de dépôt de pavés de colle sur un élément de protection et une étape de mise en place de l'élément de protection pour transférer les pavés de colle sur les zones de la serviette périodique à encoller, les pavés de colle sur l'élément de protection étant disposés pour venir recouvrir la zone centrale et une partie des volets latéraux et une zone antérieure et une zone postérieure de la serviette périodique.

D'autres caractéristiques et avantages de la présente invention apparaîtront plus clairement à la lecture de la description d'un mode de réalisation, ci-après faite en référence aux dessins annexés dans lesquels :
- la figure 1 représente une vue côté linge d'une serviette périodique selon l'invention avant sa mise en place par l'utilisatrice sur le linge de corps ;
- la figure 2 représente une vue côté linge de la serviette périodique avant utilisation.

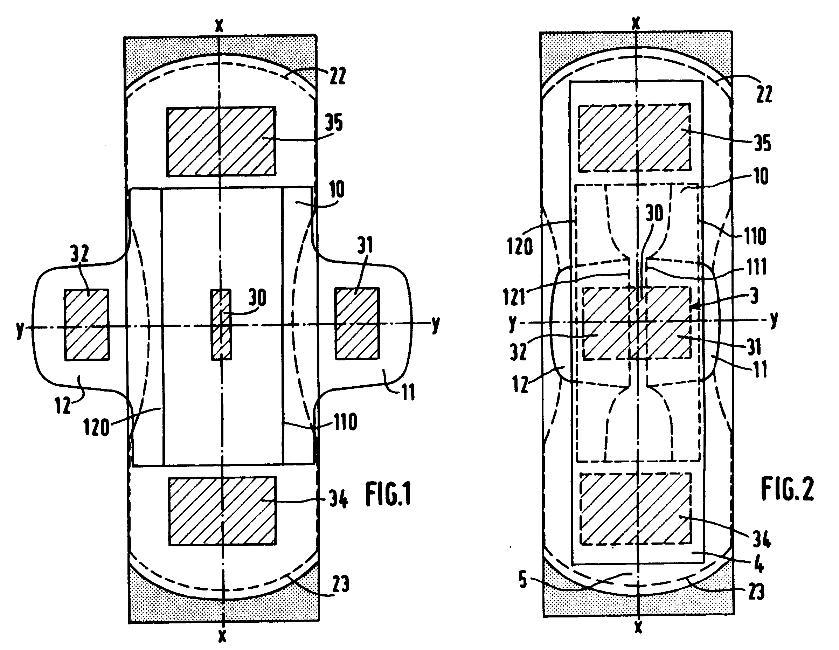

La serviette périodique ou similaire, en particulier pour l'hygiène féminine, représentée à la figure 1 comprend un élément tampon absorbant les fluides disposé entièrement à l'intérieur d'une enveloppe dont au moins la partie de réception des fluides opposée à la partie visible sur la figure 1 est perméable aux fluides.

Cette enveloppe est fermée par les lignes de soudure (22, 23) en forme d'arrondi aux extrémités de la serviette périodique, et le long de la serviette. L'élément tampon de la serviette peut éventuellement être préformé, par exemple de forme générale bi-concave, comme cela est bien connu dans le domaine des serviettes périodiques.

L'élément tampon peut être encore préformé en étant sensiblement rectangulaire et en se terminant par une partie plus étroite à l'arrière, comme cela est également bien connu. Selon le mode de réalisation représenté, une bande en matériau imperméable (10) est rapportée sur la face côté linge de la serviette. Elle comporte des volets (11, 12) latéraux s'étendant transversalement depuis la partie centrale de la serviette périodique. Selon un autre mode de réalisation connu, les volets latéraux peuvent être réalisés à partir de l'enveloppe, sans pièce rapportée.

Ces volets latéraux sont d'une dimension suffisante pour pouvoir être rabattus extérieurement autour du linge de corps porté par la personne et comportent des moyens de solidarisation amovible (31, 32) pour permettre une fixation des volets extérieurement sur le linge de corps. On obtient ainsi une protection complète du linge de corps.

La partie centrale de la serviette tournée vers le linge de corps comporte également à ses extrémités antérieure et postérieure des moyens de solidarisation constitués par des pavés adhésifs, respectivement (35, 34), avec le linge de corps. Selon un mode de réalisation particulièrement préféré, une bande adhésive centrale (30) de faible largeur au niveau des volets est également prévue dans la partie centrale de la bande imperméable (10). Les moyens de solidarisation sont de préférence constitués de pavés adhésifs sensibles à la pression obtenus par une enduction appropriée, un transfert ou tout autre procédé. Ils peuvent également être constitués à partir d'un papier adhésivé double face.

Cette bande centrale (30) vient se coller sur la face intérieure du linge de corps tandis qu'un des pavés adhésifs (31, 32) vient se coller sur la face extérieure du linge de corps, l'autre pavé venant par exemple se coller sur le volet collé au linge de corps.

La figure 2 permet de comprendre le procédé de fabrication de la serviette selon l'invention.

Lorsque l'élément absorbant disposé dans une enveloppe a été rendu solidaire d'une bande en matériau imperméable (10) qui est découpée de façon à former les volets latéraux (11, 12), comme représenté à la figure 1, le processus de fabrication comporte l'étape de pliage des volets suivante. Les volets sont rabattus vers le tampon autour de premières lignes de pliage longitudinales (110, 120) situées soit aux bords longitudinaux de l'enveloppe, soit légèrement en retrait comme cela est représenté sur le mode de réalisation illustré. Ensuite les volets sont repliés sur eux-mêmes autour de deuxièmes lignes de pliage (111, 121) longitudinales. Les lignes de pliage (111, 121) de ces volets latéraux (11, 12) sont situées entre les deux premières lignes de pliage et à proximité de la ligne (5) formant l'axe de symétrie longitudinal de la serviette. Elles sont espacées l'un de l'autre de façon à laisser entre elles vers l'intérieur une bande de faible largeur.

Les volets (11, 12) ainsi pliés sont maintenus en place et amenés à un poste de travail où une bande en papier siliconé (4), préalablement enduite de trois pavés, dont deux (34, 35), sont disposés respectivement aux extrémités antérieure et postérieure de la bande et un pavé (3) est situé dans la partie centrale de la bande. La bande de papier siliconé est ensuite plaquée sur la serviette, ayant les volets (11, 12) pliés, de façon que l'adhésif (3) de la partie centrale se dépose d'une part sur les faces externes de la partie pliée des volets pour former les pavés adhésifs (32, 31) et, d'autre part sur la partie centrale de la bande (10) pour former le pavé adhésif longitudinal (30).

Les volets (11, 12) ont une taille telle qu'une fois repliés et recouverts de la bande siliconée (4), leur extrémité dépasse latéralement de cette bande siliconée pour permettre à l'utilisatrice de tirer dessus et ainsi décoller plus facilement la bande siliconée (4).

En utilisation, lorsque l'utilisatrice enlève la bande en papier siliconé (4), l'adhésif transféré (30, 31, 32) formant une couche continue assure le maintien en place des volets (11, 12) contre la partie centrale.

Ceci permet éventuellement à l'utilisatrice de se servir de la serviette périodique, sans déployer les volets, comme une serviette normale si elle ne veut pas que les volets entourent son linge de corps. Dans le cas où elle veut entourer le linge de corps, elle tire sur les extrémités latérales des volets (11, 12) de façon à désolidariser les volets de la bande centrale d'adhésif (30) et se retrouver avec une serviette conformée selon la figure 1.

L'utilisation d'une telle serviette correspond ensuite à l'utilisation de l'art antérieur.

On comprend donc l'intérêt d'une telle serviette, tant sur le plan de la flexibilité d'utilisation pour l'utilisatrice que sur le plan de sa facilité de mise en oeuvre. En outre, sur le plan industriel, le procédé de fabrication se distingue du procédé antérieur par le fait que dans le procédé antérieur il était nécessaire, soit de déposer des pavés spéciaux sur les volets latéraux après la mise en place de la bande siliconée, soit de revêtir les deux faces de la bande siliconée d'un adhésif de façon à former sur une première face les pavés de la partie centrale et sur l'autre face les pavés des volets latéraux et, ensuite, de replier les volets de façon à ce que les parties pliées des volets viennent se coller sur les pavés adhésifs formés sur la face de la bande siliconée orientée vers l'extérieur de la serviette périodique.

Dans un tel procédé la serviette périodique obtenue ne peut être utilisée comme une serviette périodique normale car il faut obligatoirement désolidariser les volets de la bande siliconée pour pouvoir ensuite enlever celle-ci.

En outre, du point de vue de la fabrication, il est difficile de manipuler une bande siliconée enduite d'adhésif sur les deux faces et de plier les volets de façon à ce que les pliures correspondent exactement aux endroits où l'adhésif doit se coller, ce qui rend le procédé de fabrication très complexe par rapport au procédé de l'invention qui est bien plus simple à mettre en oeuvre.

La disposition des volets sous la bande siliconée procure en outre une excellente stabilité du pliage facilitant les opérations ultérieures de conditionnement, par exemple pliage de la serviette en trois et mise en place dans une enveloppe pochette individuelle.

## Revendications

1. Serviette périodique ou similaire, en particulier pour l'hygiène féminine, comprenant un élément tampon absorbant les fluides disposé à l'intérieur d'une enveloppe dont au moins la partie de réception des fluides est perméable aux fluides, des volets latéraux (11, 12) étant prévus de part et d'autre de l'élément tampon pour améliorer la tenue et l'étanchéité latérale, lesdits volets latéraux étant rabattus sur l'élément tampon autour de premières lignes de pliage (110, 120) et repliés sur eux-mêmes vers l'extérieur autour de deuxièmes lignes de pliage (111, 121), les plis (110, 120) étant espacés l'un de l'autre de façon à laisser apparaître une bande centrale (30) de l'enveloppe, lesdits volets étant de dimension suffisante pour pouvoir être rabattus extérieurement autour du linge de corps, tel qu'un slip, porté par l'utilisatrice, caractérisée en ce que trois moyens adhésifs distincts de solidarisation sont prévus, un premier moyen adhésif sous la forme d'une couche continue (30, 31, 32) prévue sur la face externe des volets repliés et sur la bande centrale de l'enveloppe et des second et troisième moyens adhésifs (34, 35) prévus aux extrémités antérieure et postérieure de l'enveloppe, et en ce que des moyens de protection (4) recouvrent lesdits moyens de solidarisation.

2. Serviette périodique selon la revendication 1, caractérisée en ce que les moyens de protection (4) sont constitués par une bande de papier siliconé recouvrant lesdits moyens adhésifs (30, 31, 32, 34, 35).

3. Serviette périodique selon la revendication 1, caractérisée en ce que les volets latéraux (11, 12) sont constitués d'un matériau imperméable au fluide revêtu d'une matière absorbante.

4. Procédé de fabrication de serviettes périodiques selon l'une des revendications précédentes, caractérisé en ce qu'il comporte des étapes de formation de l'élément tampon associé avec un élément imperméable (10) faisant face au vêtement et pourvu dans la zone centrale de volets latéraux (11, 12), caractérisé en ce qu'il comporte en outre :
- une étape de pliage des volets autour des lignes de pliage longitudinales sur la zone centrale d'une part sur eux-mêmes d'autre part, en ménageant une bande centrale (30) non recouverte par les volets (11, 12) ;
- une étape de dépôt de trois pavés de colle, un premier pavé (30, 31, 32) recouvrant la zone centrale et une portion de surface des volets tournés vers l'extérieur de la serviette périodique et un second et troisième pavés (34, 35) disposés respectivement aux extrémités antérieure et postérieure de l'enveloppe ;
- une étape de mise en place d'un élément de protection (4) des parties encollées.

5. Procédé selon la revendication précédente, caractérisé en ce que l'étape de dépôt de pavés de colle consiste en l'application d'une bande, telle que du papier siliconé, recouverte d'une couche d'adhésif, ladite couche d'adhésif adhérant plus fortement à la matière constituant la serviette périodique que celle de ladite bande, et ladite bande formant l'élément de protection (4).

## Claims

1. Sanitary towel or the like, in particular for feminine hygiene, comprising a pad element absorbing fluids disposed inside an envelope of which at least the part receiving fluids is permeable to fluids, side flaps (11, 12) being provided either side of the pad element for improving the fit and lateral fluid tightness, the said side flaps being folded over onto the pad element about the first fold lines (110, 120) and being folded onto themselves outwards about the second fold lines (111, 121), the folds (110, 120) being spaced from each other so as to reveal a central strip (30) of the envelope, the said side flaps being of a sufficient size so that they can be folded over externally around the underwear, such as pants worn by the user, characterized in that three distinct adhesive means of attachment are provided, a first adhesive means in the form of a continuous layer (30, 31, 32) provided on the outer face of the folded flaps and on the central strip of the envelope and second and third adhesive means (34, 35) provided at the front and rear ends of the envelope, and in that means of protection (4) cover the said means of attachment.

2. Sanitary towel according to claim 1, characterized in that the means of protection (4) consist of a strip of siliconized paper covering the said adhesive means (30, 31, 32, 34, 35).

3. Sanitary towel according to claim 1, characterized in that the side flaps (11, 12) consist of a material impermeable to fluid covered with an absorbent material.

4. Process for manufacturing sanitary towels according to one of the preceding claims, characterized in that it comprises stages of forming the pad element associated with an impermeable element (10) facing the clothing and provided in the central area with side flaps (11, 12), characterized in that it additionally comprises :
- a stage of folding the flaps onto the central area about longitudinal fold lines, on the one hand onto the central area and on the other hand onto themselves, while providing a central strip (30) not covered by the flaps (11, 12);
- a stage of depositing three pads of adhesive, a first pad (30, 31, 32) covering the central zone and a portion of the surface of the flaps turned outwards from the sanitary towel and second and third pads (34, 35) disposed at the front and rear ends respectively of the envelope;
- a stage of putting an element (4) in place for protecting the adhesive coated parts.

5. Process according to the preceding claim, characterized in that the stage of depositing the pads of adhesive consists of applying a strip, such as a strip of siliconized paper, covered with a layer of adhesive, the said layer of adhesive adhering more strongly to the material constituting the sanitary towel than to that of the said strip, and the said strip forming the protective element (4).

## Patentansprüche

1. Damenbinde oder dergleichen, insbesondere für die weibliche Hygiene, mir einem flüssigkeitsabsorbierenden Tamponelement, das im Inneren einer Hülle angeordnet ist, von der mindestens der flüssigkeitsaufnehmende Abschnitt flüssigkeitsdurchlässig ist, wobei Seitenteile (11, 12) beidseitig zu dem Tamponelement vorgesehen sind, um deren Halt und seitliche Abdichtung zu verbessern, wobei die Seitenteile auf das Tamponelement um erste Faltinien (110, 120) gefaltet sind und in Auswärtsrichtung um zweite Faltlinien (111, 121) auf sich selbst zurückgefaltet sind, wobei die Faltungen (110, 120) zueinander so beabstandet sind, daß sie ein zentrales Band (30) der Hülle freilassen, wobei die Seitenteile ausreichend große Abmessungen haben, um außen um von der Benutzerin getragene Unterwäsche wie z.B. einen Slip geschlagen zu werden, dadurch gekennzeichnet, daß drei verschiedene Klebeverbindungsmittel vorhanden sind, wobei ein erstes Klebeverbindungsmittel in der Form einer kontinuierlichen Schicht (30, 31, 32) auf der Außenseite der zurückgefalteten Seitenteile vorgesehen ist und auf dem zentralen Band (30) der Hülle und zweite und dritte Klebeverbindungsmittel (34, 35) an den vorderen und hinteren Enden der Hülle vorgesehen sind, und daß Schutzmittel (4) die Verbindungsmittel bedecken.

2. Damenbinde nach Anspruch 1, dadurch gekennzeichnet, daß die Schutzmittel (4) von einem Silikonpapierstreifen gebildet sind, der die besagten Klebeverbindungsmittel (30, 31, 32, 34, 35) bedeckt.

3. Damenbinde nach Anspruch 1, dadurch gekennzeichnet, daß die Seitenteile (11,12) von einem flüssigkeitsundurchlässigen Material gebildet sind, das mit einem absorbierenden Stoff versehen ist.

4. Verfahren zum Herstellen von Damenbinden nach einem der vorhergehenden Ansprüche, gekennzeichnet durch die Bildung eines Tamponelementes, das einem undurchlässigen Element (10) zugeordnet ist, welches der Kleidung zugewandt und in dem zentralen Bereich mit Seitenteilen (11, 12) versehen ist, gekennzeichnet durch die folgenden weiteren Schritte:
- Falten der Seitenteile um längs verlaufende Faltlinien auf den zentralen Bereich einerseits und auf sich selbst andererseits, wobei ein von den Seitenteilen (11, 12) nicht bedecktes zentrales Band (30) freigelassen wird;
- Aufbringen von Klebestreifen, wobei ein erster Klebestreifen (30, 31, 32) den zentralen Bereich sowie einen Flächenabschnitt der zur Außenseite der Damenbinde gewandten Seitenteile bedeckt und ein zweiter und dritter Klebestreifen (34, 35) jeweils an den vorderen und hinteren Enden der Hülle angeordnet sind;
- Vorsehen eines Schutzelementes (4) auf den Klebeabschnitten.

5. Verfahren nach dem vorhergehenden Anspruch, dadurch gekennzeichnet, daß das Aufbringen von Klebestreifen aus dem Anbringen eines mit einer Klebeschicht versehenen Bandes wie z.B. Silikonpapier besteht, wobei die Klebeschicht an dem Material der Damenbinde stärker als an dem des Bandes haftet und dieses Band das Schutzelement (4) bildet.
